# EUROPÄISCHE PATENTSCHRIFT

(11) **EP 0 585 627 B1**
(45) Veröffentlichungstag und Bekanntmachung des Hinweises auf die Patenterteilung: **03.01.1996**
(21) Anmeldenummer: 93112351.7
(22) Anmeldetag: 02.08.1993
(51) Int. Cl.: C25B 3/08, C25B 11/04

(54) **Verfahren zur Herstellung von Perfluoralkylsulfonylfluoriden sowie Elektroden zur Durchführung des Verfahrens**
A process for preparing perfluoroalkylsulfonylfluorides and electrodes for carrying out said process
Procédé pour la préparation de perfluroalkylfluoriures ainsi que d'électrodes pour la réalisation du procédé

(30) Priorität: 13.08.1992 DE 4226758
(43) Veröffentlichungstag der Anmeldung: 09.03.1994
(73) Patentinhaber: BAYER AG, D-51368 Leverkusen (DE)
(72) Erfinder: Bulan, Andreas Dipl.-Ing., D-40764 Langenfeld (DE); Weber, Rainer Dr., D-51519 Odenthal (DE); Muziol, Zbigniew Dipl.-Ing, D-51377 Leverkussen (DE); Schlecker, Hartmut, Dr., D-51373 Leverkusen (DE); Moretto, Hans-Heinrich Dr., D-51375 Leverkusen (DE)

(56) Entgegenhaltungen:
- EP-A- 0 451 589
- FR-A- 2 225 399
- FR-A- 2 243 274

## Beschreibung

Die Erfindung betrifft ein Verfahren zur Herstellung von Perfluoralkylsulfonylfluoriden durch elektrochemische Fluorierung der entsprechenden Alkylsulfonylhalogeniden.

Die Herstellung von fluorierten Verbindungen durch elektrochemische Fluorierung erfolgt gemäß E. Hollitzer und P. Satori, Chem.-Ing.-Tech. 58 (1986), Nr. 1, 31-38, durch anodische Oxidation in einem fluoridhaltigen Lösungsmittel. Bei den Verfahren kommen als Anodenmaterialien Nickel, Kohlenstoff und Platin zum Einsatz. Wenn als Lösungsmittel Fluorwasserstoff verwendet wird, werden vorzugsweise Nickelanoden eingesetzt. Bei den Verfahren werden perfluorierte Verbindungen erhalten.

Werden Alkylsulfonylhalogenide der Formel CₙH₂ₙ₊₁SO₂X mit n = 6-10 und X = F, Cl, Br, J in Fluorwasserstoff an Nickelelektroden elektrochemisch fluoriert, entstehen Perfluoralkylsulfonylfluoride, Cₙf₂ₙ₊₁SO₂F. Ein Nachteil dieses Verfahrens zur Herstellung von Perfluoralkylsulfonylfluoriden ist die erhebliche Korrosion der Anoden. Diese Korrosion führt zu Problemen beim Betrieb der Elektrolysezellen durch Korrosionsprodukte des Nickels, die die Elektrodenzwischenräume zusetzen und Ventile und Rohrleitungen verstopfen. Dadurch ist eine langandauernde, kontinuierliche elektrochemische Fluorierung nicht möglich, was u.a. die Wirtschaftlichkeit des Verfahrens stark beeinträchtigt.

Aufgabe war es daher, ein Verfahren zur Herstellung von Perfluoralkylsulfonylfluoriden durch elektrochemische Fluorierung zur Verfügung zu stellen, bei dem die genannten Nachteile nicht auftreten.

Es wurde überraschenderweise gefunden, daß die genannten Nachteile nicht auftreten, wenn die Anoden aus Nickel mit kolumnarer Struktur bestehen oder mit Nickel einer solchen Struktur beschichtet sind.

Gegenstand der Erfindung ist ein Verfahren zur Herstellung von Perfluoralkylsulfonylfluoriden der Formel CₙF₂ₙ₊₁SO₂F mit n = 6-10 durch elektrochemische Fluorierung von Alkylsulfonylhalogeniden der allgemeinen Formel CₙH₂ₙ₊₁SO₂X mit n = 6-10 und X = F, Cl, Br oder J in Fluorwasserstoff, welches dadurch gekennzeichnet ist, daß Elektroden eingesetzt werden, die aus Nickel mit kolumnarer Struktur bestehen oder die mit Nickel mit kolumnarer Struktur beschichtet sind.

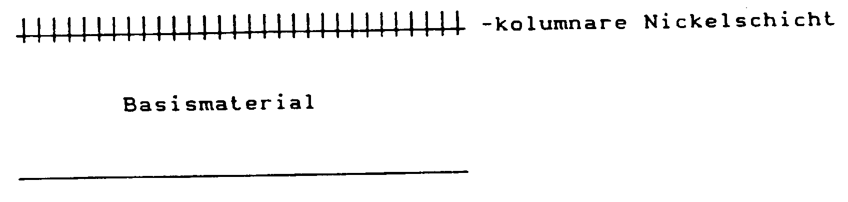

Als Elektrodenmaterial kann bei Elektroden, die mit kolumnarem Nickel beschichtet sind, neben Nickel auch ein anderes Metall, wie z.B. Eisen oder Kupfer, eingesetzt werden.

Nickel mit kolumnarer Struktur wird z.B. durch elektrolytisches Abscheiden von Nickel auf einem Basismaterial erhalten (Praktische Galvonotechnik, Ein Lehr- und Handbuch, 4. Aufl. 1984, Eugen G. Leuze Verlag, Saulgau).

Bevorzugt werden im erfindungsgemäßen Verfahren Octylsulfonylhalogenide der Formel C₈H₁₇SO₂X mit X=F, Cl oder Br zu Perfluoroctylsulfonylfluorid elektrochemisch fluoriert.

Werden bei der Herstellung von Perfluoralkylsulfonylfluoriden der Formel CₙF₂ₙ₊₁SO₂F mit n = 6-10 Anoden aus Nickel mit kolumnarer Struktur oder Anoden, die mit Nickel kolumnarer Struktur beschichtet sind, eingesetzt, wird keine Korrosion beobachtet. Somit kann die elektrochemische Fluorierung langandauernd und kontinuierlich betrieben werden. Stillstandzeiten, in denen Elektrodenpakete gewechselt werden müssen, weil sie durch die Korrosionsprodukte des Nickels zugesetzt sind, entfallen. Außerdem verbessert die längere Standzeit der Anoden die Wirtschaftlichkeit des Verfahrens.

Die elektrochemische Fluorierung wird üblicherweise unter den folgenden Bedingungen durchgeführt:

Die für die elektrochemische Fluorierung verwendeten Elektrolysezellen bestehen aus Nickel oder einem anderen, gegenüber Fluorwasserstoff korrosionsbeständigen Material, wie beispielsweie perfluorierte Kunststoffe. Die Kathoden bestehen aus Nickel oder Eisen. Als Anoden werden die erfindungsgemäßen Elektroden eingesetzt. Die Abstande der Elektroden betragen normalerweise 2 bis 5 mm. Die Elektrolyttemperatur liegt üblicherweise zwischen 0°C und 20°C. Sie kann aber auch, wie in der DE-A 2 442 106 beschrieben, zwischen 20°C und 50°C liegen. Ausführliche Einzelheiten, die Aufbau und Betriebsbedingungen der Elektrolysezellen betreffen, sind der Literatur zu entnehmen.

In einer Elektrolysezelle werden Fluorwasserstoff und das zu fluorierende Alkylsulfonylhalogenid vorgelegt. Im Anschluß daran wird eine konstante Zellspannung von 4 bis 6 V angelegt und die zu fluorierende Verbindung entsprechend der geflossenen Strommenge und der gegebenen Stöchiometrie (siehe allgemeine Reaktionsgleichung) kontinuierlich oder diskontinuierlich nachdosiert: 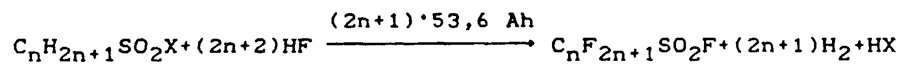 X = F, Cl, Br, J n = 6-10

Fluorwasserstoff wird nach Verbrauch zudosiert. Das fluorierte Produkt wird in bestimmten Zeitabständen diskontinuierlich aus der Zelle entfernt.

Die Erfindung soll anhand der nachfolgenden Beispiele näher erläutert werden.

### Beispiel 1 (Stand der Technik)

### Elektrochemische Fluorierung von Octylsulfonylflourid an Nickel-Blech-Elektroden mit polykristalliner Struktur

Durch elektrochemische Fluorierung von Octylsulfonylfluorid (C₈H₁₇SO₂F) wird Perfluoroctylsulfonylfluorid (C₈F₁₇SO₂F) hergestellt. Dazu wird die Elektrolysezelle mit 26,5 kg Fluorwasserstoff gefüllt. Die Elektrolyttemperatur beträgt 10°C. Das Octylsulfonylfluorid wird nach gegebener Stöchiometrie kontinuierlich zugegeben.

Die Anoden und Kathoden bestehen aus polykristallinem Nickel-Blech (2 mm dick). Die Maße einer Anode sind 25 cm in der Breite und 20 cm in der Höhe. Die Anodenfläche beträgt 9750 cm.

Bei einer Spannung von durchschnittlich 5 V werden 3,1 kg Octylsulfonylfluorid mit einer Ladungsmenge von 15.142 Ah in 1532 Betriebsstunden umgesetzt. Der Nickelverlust beträgt während der Versuchszeit 499 g; dies entspricht 32,9 mg pro Amperestunde.

### Beispiel 2 (Stand der Technik)

### Elektrochemische Fluorierung an polykristallinem Nickel-Schaum

Durch elektrochemische Fluorierung von Octylsulfonylfluorid (C₈H₁₇SO₂F) wird Perfluoroctylsulfonylflourid (C₈F₁₇SO₂F) hergestellt. Dazu wird die Elektrolysezelle mit 650 g Fluorwasserstoff gefüllt. Die Elektrolyttemperatur beträgt 15°C. Das Octylsulfonylfluorid wird nach gegebener Stöchiometrie zugegeben.

Die Anoden bestehen aus polykristallinem Nickel-Schaum der Fa. NiTech, Fontenay-sous-Bois Cedex, Frankreich (Typ MN 045 0200 050), die Kathoden aus polykristallinem Nickel-Blech. Die Maße einer Anode betragen: 7 cm hoch, 5 cm breit, 0,2 cm dick. Die geometrische Gesamtanodenfläche beträgt 271 cm.

Bei einer Spannung von durchschnittlich 5 V wird versucht, 20 g Octylsulfonylflourid umzusetzen. Nach 191 Stunden und 84,7 Ah sind die Anoden derart stark korrodiert, daß der Versuch abgebrochen werden muß, da die Elektroden zum Teil aufgelöst sind.

Der Nickelverlust beträgt während der Versuchszeit 3,5 g, dies entspricht 41,3 mg Nickel pro Amperestunde.

### Beispiel 3 (erfindungsgemäß)

### Elektrochemische Fluorierung an kolumnarem Nickel

Durch elektrochemische Fluorierung von Octylsulfonylfluorid (C₈H₁₇SO₂F) wird Perfluoroctylsulfonylflourid (C₈F₁₇SO₂F) hergestellt. Dazu wird die Elektrolysezelle mit 27 kg Fluorwasserstoff gefüllt. Die Elektrolyttemperatur beträgt 10°C. Das Octylsulfonylfluorid wird nach gegebener Stöchiometrie zugegeben.

Die Anoden bestehen aus Nickel-Schaum der Fa. DUNLOP, Coventry, Großbritannien (Retimet), wobei das Nickel kolumnare Struktur besitzt. Die Maße einer Anode betragen: 20 cm x 25 cm x 1 cm. Das Elektrodenpaket besteht aus 7 Anoden und 8 Kathoden. Die geometrische Gesamtanodenfläche beträgt 6.825 cm.

Bei einer Spannung von durchschnittlich 5 V werden 106,1 kg Octylsulfonylflourid mit 493.326 Ah in 19.782 Stunden umgesetzt. Nach Beendigung des Versuchs kann keine Elektrodenkorrosion festgestellt werden.

## Patentansprüche

1. Verfahren zur Herstellung von Perfluoralkylsulfonylfluoriden der Formel CₙF₂ₙ₊₁SO ₂F mit n = 6-10 durch elektrochemische Fluorierung von Alkylsulfonylhalogeniden der Formel CₙH₂ₙ₊₁SO₂X mit n = 6-10 und mit X = F, Cl, Br oder J in Fluorwasserstoff, dadurch gekennzeichnet, daß Elektroden, vorzugsweise Anoden, eingesetzt werden, die aus Nickel mit kolumnarer Struktur bestehen oder die mit Nickel mit kolumnarer Struktur beschichtet sind.

2. Verfahren gemäß Anspruch 1, dadurch gekennzeichnet, daß Octylsulfonylhalogenide der Formel C₈H₁₇SO₂X mit X = F, Cl oder Br in Fluorwasserstoff elektrochemisch unter Verwendung von Elektroden, die aus Nickel mit kolumnarer Struktur bestehen oder die mit Nickel mit kolumnarer Struktur beschichtet sind, fluoriert werden.

## Claims

1. Process for the production of perfluoroalkylsulphonyl fluorides of the formula CₙF₂ₙ₊₁SO₂F with n = 6-10 by electrochemical fluorination of alkylsulphonyl halides of the formula CₙH₂ₙ₊₁SO₂X with n = 6-10 and with X = F, Cl, Br or I in hydrogen fluoride, characterised in that electrodes, preferably anodes, are used which consist of nickel with a columnar structure or which are coated with nickel with a columnar structure.

2. Process according to claim 1, characterised in that octylsulphonyl halides of the formula C₈H₁₇SO₂X with X = F, Cl or Br are electrochemically fluorinated in hydrogen fluoride using electrodes which consist of nickel with a columnar structure or which are coated with nickel with a columnar structure.

## Revendications

1. Procédé de préparation des fluorures de perfluoralkylsulfonyle de formule CₙF₂ₙ₊₁SO₂F dans laquelle n = 6 à 10 par fluoration électrochimique des halogénures d'alkylsulfonyle de formule CₙH₂ₙ₊₁SO₂X dans laquelle n = 6 à 10 et X = F, Cl, Br ou I dans le fluorure d'hydrogène, caractérisé en ce l'on utilise des électrodes, surtout des anodes, qui consistent en nickel à structure columnaire ou qui sont revêtues de nickel à structure columnaire.

2. Procédé selon la revendication 1, caractérisé en ce que l'on soumet des halogénures d'octylsulfonyle de formule C₈H_{I7}SO₂X dans laquelle X = F, Cl ou Br à fluoration électrochimique dans le fluorure d'hydrogène à l'aide d'électrodes consistant en nickel à structure columnaire ou revêtues de nickel à structure columnaire.
